# EUROPÄISCHE PATENTSCHRIFT

(11) **EP 2 802 283 B1**
(45) Veröffentlichungstag und Bekanntmachung des Hinweises auf die Patenterteilung: **07.03.2018**
(21) Anmeldenummer: 13701223.3
(22) Anmeldetag: 11.01.2013
(51) Int. Cl.: A61B 90/90, A61B 90/98

(54) **AUFBEWAHRUNGS- UND/ODER TRANSPORTBEHÄLTER FÜR MEDIZINISCHE INSTRUMENTE UND VERFAHREN ZUM ERFASSEN UND ÜBERTRAGEN VON DATEN MEDIZINISCHER INSTRUMENTE**
STORAGE AND/OR TRANSPORT CONTAINER FOR MEDICAL INSTRUMENTS AND METHOD FOR DETECTING AND TRANSFERRING DATA OF MEDICAL INSTRUMENTS
RÉCEPTACLE DE RANGEMENT ET/OU DE TRANSPORT POUR INSTRUMENTS MÉDICAUX ET PROCÉDÉ PERMETTANT DE DÉTECTER ET DE TRANSFÉRER DES DONNÉES RELATIVES AUXDITS INSTRUMENTS MÉDICAUX

(30) Priorität: 13.01.2012 DE 102012000860
(43) Veröffentlichungstag der Anmeldung: 19.11.2014
(73) Patentinhaber: Karl Storz SE & Co. KG, 78532 Tuttlingen (DE)
(72) Erfinder: DRUZINIC-FIEBACH, Rok, 16548 Glienicke/Nordbahn (DE)
(74) Vertreter: Witte, Weller & Partner Patentanwälte mbB
(86) Internationale Anmeldenummer: PCT/EP2013/050458
(87) Internationale Veröffentlichungsnummer: WO 2013/104743

(56) Entgegenhaltungen:
- DE-U1-202011 050 001
- US-A1- 2006 119 481
- US-A1- 2006 208 072
- US-A1- 2006 244 593

## Beschreibung

Die Erfindung betrifft einen Aufbewahrungs- und/oder Transportbehälter für medizinische Instrumente, mit einem Behälter zur Aufnahme der medizinische Instrumente und einer Vorrichtung zum Erfassen und Übertragen von Daten der medizinischen Instrumente, die eine Steuereinheit, eine Ausleseeinheit und zumindest ein an zumindest einem medizinischen Instrument angebrachtes erstes Kennzeichnungselement aufweist, wobei die Steuereinheit mit der Ausleseeinheit in Verbindung steht und mit der Ausleseeinheit, ausgelöst durch Signale, Daten austauscht und diese weiterverarbeitet, speichert und/oder an ein an dem Behälter angebrachtes zweites Kennzeichnungselement weiterleitet, wobei die Ausleseeinheit Daten mit dem zumindest einen ersten Kennzeichnungselement austauscht und an die Steuereinheit weiterleitet.

Ein solcher Aufbewahrungs- und/oder Transportbehälter für medizinische Instrumente mit einem Behälter zur Aufnahme der medizinische Instrumente und einer Vorrichtung zum Erfassen und Übertragen von Daten der medizinischen Instrumente ist aus US 2006/0244593 A1 bekannt.

Ein Aufbewahrungs- und/oder Transportbehälter für medizinische Instrumente mit einer Vorrichtung zum Erfassen und Übertragen von Daten der medizinischen Instrumente der eingangs genannten Art kann im Sinne der vorliegenden Erfindung zur Aufnahme, Überwachung und Nachverfolgung von medizinischen Instrumenten bei Prozessabläufen innerhalb eines Krankenhauses verwendet werden.

Hierzu werden die Aufbewahrungs- und/oder Transportbehälter, im Folgenden auch als Krankenhaussiebe bezeichnet, gekennzeichnet, um sie bei den innerhalb eines Krankenhauses ablaufenden Prozessen nachverfolgen zu können. Die Kennzeichnung der Krankenhaussiebe wird typischerweise durch auf den Krankenhaussieben aufgebrachten Data Matrix Codes oder durch sogenannte RFID-Transponder gewährleistet. Durch diese Kennzeichnungselemente, die typischerweise an den Krankenhaussieben angebracht sind, ist somit durch eine dementsprechende Ausleseinfrastruktur innerhalb eines Krankenhauses eine gute Nachverfolgung der Krankenhaussiebe gewährleistet.

In diesem Zusammenhang und den noch folgenden Beschreibungen steht die Abkürzung RFID für Radio Frequency Identification Device und bezeichnet allgemein eine Vorrichtung zur drahtlosen Übertragung von spezifischen Daten.

Auch die medizinischen Instrumente, die sich in einem solchen Krankenhaussieb befinden, sind üblicherweise mit solchen RFID-Transpondern ausgerüstet, um eine kontaktlose Erfassung der medizinischen Instrumente im Krankenhaussieb zu gewährleisten. Die Erfassung der medizinischen Instrumente im Krankenhaussieb kann durch eine am bzw. im Krankenhaussieb angebrachte Ausleseeinheit gewährleistet werden, die im Anschluss die von den medizinischen Instrumenten ermittelten Daten an die Ausleseinfrastruktur des Krankenhauses übermittelt.

Hierbei tauschen die an den medizinischen Instrumenten angebrachten ersten Kennzeichnungselemente und die Ausleseeinheit, die an oder in dem Krankenhaussieb angebracht sind, entweder dauerhaft Daten aus, oder der Datenaustausch wird durch ein Signal aktiviert. Demnach wird der Auslesevorgang entweder manuell durch beispielsweise das Drücken eines Knopfes am Krankenhaussieb aktiviert oder der Auslesevorgang wird durch ein externes Signal vermittelt, das beispielsweise durch die externe Ausleseinfrastruktur des Krankenhauses an die Ausleseeinheit des Krankenhaussiebes übermittelt werden kann.

Die zuvor genannten Ausleseprozesse haben den Nachteil, dass sie entweder manuell in Gang gesetzt werden und/oder sehr energieaufwändig sind, da die aktiven Auslese- und Sendekomponenten des Krankenhaussiebes für lange Zeit aktiv, entweder mit den medizinischen Instrumenten und/oder mit der externen Ausleseinfrastruktur des Krankenhauses, Daten austauschen.

Darüber hinaus ist nachteilig, dass auch dann Daten von den medizinischen Instrumenten ausgelesen werden, wenn sich deren biologische, physikalische oder chemische Eigenschaften nicht verändert haben, was zu einer unnötigen Belastung der Dateninfrastruktur eines Krankenhauses führen kann.

Der Erfindung liegt daher die Aufgabe zugrunde, einen Aufbewahrungs- und/oder Transportbehälter für medizinische Instrumente mit einer Vorrichtung zum Erfassen und Übertragen von Daten der eingangs genannten Art dahingehend weiterzubilden, dass eine effiziente und bedarfsgerechte Übermittlung von Daten der medizinischen Instrumente und/oder des Krankenhaussiebes gewährleistet werden kann.

Erfindungsgemäß wird diese Aufgabe hinsichtlich des eingangs genannten Aufbewahrungs- und/oder Transportbehälters für medizinische Instrumente mit einem Behälter zur Aufnahme der medizinische Instrumente und einer Vorrichtung zum Erfassen und Übertragen von Daten der medizinischen Instrumente dadurch gelöst, dass die Vorrichtung eine Sensoreinheit aufweist, die das zumindest eine medizinische Instrument durch eine an ihm durchgeführte Messung zumindest einer Messgröße erfasst, und mit der Steuereinheit Signale austauscht, wobei die ausgetauschten Signale den Austausch von Daten zwischen der Ausleseeinheit und dem zumindest einen ersten Kennzeichnungselement in Gang setzt, wobei die Ausleseeinheit die ausgetauschten Daten an die Steuereinheit übermittelt.

Die aus US 2006/0244593 A1 bekannte Vorrichtung und der darin beschriebene Aufbewahrungs- und/oder Transportbehälter für medizinische Instrumente geht von dem Konzept aus, dass der Auslesevorgang der den medizinischen Instrumenten zugeordneten Transpondern durch das Drücken eines Knopfes und/oder durch ein von Externen abgegebenen Signals aktiviert wird. Demnach beruht die Aktivierung des Auslesevorgangs auf einem Ereignis, das manuell entweder durch Drücken eines Knopfes oder durch ein anderes Signal hervorgerufen wird.

Die erfindungsgemäße Vorrichtung löst sich dagegen von dem Konzept, den Auslesevorgang auf manuelle Weise herbeizuführen, indem die in die Vorrichtung integrierte Sensoreinheit Messungen an zumindest einem medizinischen Instrument durchführt, und anhand der Messungen für die medizinischen Instrumente spezifische Messgrößen ermittelt, die durch eine Steuereinheit weiterverarbeitet werden und nach Abgleich der ermittelten Messgrößen mit in der Steuereinheit gespeicherten Schwellwerten gegebenenfalls zu einer Aktivierung des Auslesevorgangs des an dem zumindest einen medizinischen Instrument angebrachten zumindest einen ersten Kennzeichnungselements durch die Ausleseeinheit führt.

Die erfindungsgemäße Vorrichtung umfasst auch eine Sensoreinheit, die durch das Erreichen einer festgelegten Messgröße zumindest eines medizinischen Instruments zu einer Aktivierung des Auslesevorgangs des an dem zumindest einen medizinischen Instrument angebrachten zumindest einen ersten Kennzeichnungselements durch die Ausleseeinheit führt.

Das erfindungsgemäße Prinzip, wonach eine Ermittlung der für die medizinischen Instrumente spezifischen Daten nur im Bedarfsfall, d.h. wenn sich eine der durch die Sensoreinheit ermittelten Messgrößen der Instrumente ausreichend verändert, ermittelt wird, hat einen direkten Einfluss auf den mit der Datenerfassung in Verbindung stehenden Aufwand.

Da die Daten erfindungsgemäß lediglich im Bedarfsfall aktualisiert werden, hat die genannte Maßnahme einen vorteilhaften Einfluss auf den gesamten Datenverkehr und auf die für die Auslesevorgänge notwendige Ausleseinfrastruktur. Ferner hat das erfindungsgemäße Prinzip einen vorteilhaften Einfluss auf die durch einen Energiespeicher bereitgestellten Energieressourcen, da diese nur im Bedarfsfall, d.h. bei ausreichender Veränderung einer durch die Sensoreinheit ermittelten Messgröße relativ zu zumindest einem Schwellwert, in Anspruch genommen werden müssen.

Weiter ist von Vorteil, dass ein Defekt des zweiten am Aufbewahrungs- und/oder Transportbehälter angebrachten Kennzeichnungselements, was beispielsweise durch Alterung oder Beschädigung verursacht werden kann, schnell dadurch zu beheben ist, dass das zweite Kennzeichnungselement einfach vom Behälter entfernt und ersetzt werden kann, ohne den Aufbewahrungs- und/oder Transportbehälter öffnen zu müssen.

In einer bevorzugten Ausgestaltung weist die Steuereinheit zumindest ein Speicherelement, zumindest ein Rechnerelement, zumindest eine Übermittlungseinheit und zumindest eine Aktivierungseinheit auf, wobei die Aktivierungseinheit mit dem Speicherelement, dem Rechnerelement, der Übermittlungseinheit und der Ausleseeinheit, zur Aktivierung von Auslese-, Verarbeitungs- und/oder Übermittlungsvorgängen von Daten, verbunden ist.

Hierbei ist von Vorteil, dass alle für eine Ermittlung, Übermittlung, Auswertung, Weiterverarbeitung und Speicherung von Daten notwendigen Elemente in einer zentralen Einheit zusammengefasst sind, was den Aufwand der internen Kommunikation zwischen den Elementen auf ein Mindestmaß reduziert.

In einer weiteren Ausgestaltung ist bevorzugt, dass das zweite Kennzeichnungselement zumindest ein Datenspeicherelement und zumindest ein Sende- und/oder Empfangselement zum Austausch von Daten mit einer externen Sende- und/oder Empfangseinheit aufweist.

Hierbei ist von Vorteil, dass die für den Aufbewahrungs- und/oder Transportbehälter bzw. dessen Inhalt relevanten Daten im Datenspeicherelement des zweiten Kennzeichnungselements abgespeichert werden und diese Daten gegebenenfalls an eine externe Sende- und/oder Empfangseinheit, die bevorzugt in Form einer RFID-Infrastruktur ausgestaltet ist, übermitteln werden können.

Weiterhin ist bevorzugt, dass das zweite Kennzeichnungselement in einer weiteren Ausgestaltung zumindest einen aktiven Sender, der bevorzugt in Form eines aktiven RFID-Transponders ausgestaltet ist, aufweist.

Hierbei ist vorteilhaft, dass die Verwendung eines aktiven Senders, der bevorzugt in Form eines aktiven RFID-Transponders ausgestaltet ist, die Reichweite des Senders und damit die Konnektivität zu den externen Sende- und/oder Empfangseinheiten erhöht, was für einen ungestörten Datenaustausch vorteilhaft ist.

In einer weiteren bevorzugten Ausgestaltung weist die Ausleseeinheit zumindest ein Sende- und/oder Empfangselement auf, das mit dem zumindest einen ersten Kennzeichnungselement drahtlos Daten austauscht.

Hierbei ist von Vorteil, dass die Ausleseeinheit nicht direkt mit den medizinischen Instrumenten in Kontakt kommen muss, um etwaig benötigte Daten zu empfangen. Diese Form der kontaktfreien Datenübermittlung ist besonders hinsichtlich der Sterilisationseigenschaften der medizinischen Instrumente von entscheidender Bedeutung.

Ferner ist bevorzugt, dass die Ausleseeinheit in einer weiteren Ausgestaltung mit dem zweiten Kennzeichnungselement zumindest eine Verbindung zum Austausch von Daten aufweist.

Hierbei ist von Vorteil, dass die Ausleseeinheit direkt Daten mit dem zweiten Kennzeichnungselement austauschen kann, ohne dass diese Daten vorher in der Steuereinheit verarbeitet bzw. zwischengespeichert werden müssen.

Ferner ist es vorteilhaft, dass eine Speicherung der ermittelten Daten sowohl im zweiten Kennzeichnungselement und in der Steuereinheit möglich ist, was die Gefahr eines Datenverlustes reduziert.

In einer weiteren bevorzugten Ausgestaltung weist die Sensoreinheit zumindest einen Gewichtssensor auf, der die zumindest eine Messgröße in Form des Gewichts von zumindest einem der medizinischen Instrumente erfasst.

Des Weiteren ist bevorzugt, dass der Gewichtssensor in Form eines piezoelektrischen Elements ausgestaltet ist, wobei dieses elektrische Signale in Form von Strömen und Spannungen an die Steuereinheit übermittelt.

Hierbei ist von Vorteil, dass ein Sensor der in Form eines piezoelektrischen Elements ausgebildet ist, auf sehr einfache Weise die Existenz eines medizinischen Instruments ermitteln kann. Ferner ist an dieser Ausgestaltung vorteilhaft, dass das piezoelektrische Element zur Detektion des Gewichts der medizinischen Instrumente nicht gesondert mit Energie versorgt werden muss.

In einer weiteren bevorzugten Ausgestaltung weist der Aufbewahrungs- und/oder Transportbehälter einen Energiespeicher auf, wobei die Sensoreinheit über die Steuereinheit mit dem Energiespeicher verbunden ist.

Hierbei ist von Vorteil, dass die Energie, die in den Signalen der Sensoreinheit enthalten ist, vermittelt über die Steuereinheit an den Energiespeicher geleitet werden kann. Diese Form der Energiegewinnung ist neben der bereits beschriebenen Aktivierung des Signalauslesevorgangs durch die Ausleseeinheit eine weitere vorteilhafte Maßnahme, um die Lebensdauer des erfindungsgemäß beschriebenen Aufbewahrungs- und/oder Transportbehälters zu verlängern, da dessen Lebensdauer maßgeblich durch die noch im Energiespeicher vorhandene Menge an Energie begrenzt ist.

Ferner ist bevorzugt, dass in einer weiteren Ausgestaltung das zweite Kennzeichnungselement mit dem Energiespeicher verbindbar ist.

Diese Ausgestaltung hat den Vorteil, dass die Energiereserven des in dem zweiten Kennzeichnungselement integrierten Energiespeicherelements geschont werden.

In einer weiteren bevorzugten Ausgestaltung kann durch die Verbindung des zweiten Kennzeichnungselements mit dem Energiespeicher auf ein im zweiten Kennzeichnungselement integriertes Energiespeicherelement verzichtet werden, was die Kosten für das besagte zweite Kennzeichnungselement deutlich reduziert.

Ferner wird die eingangs genannte Aufgabe durch ein Verfahren zum Erfassen und Übertragen von Daten medizinischer Instrumente in einem Aufbewahrungs- und/oder Transportbehälter der zuvor genannten Art gelöst, das folgende Schritte aufweist:
a) Auflegen von zumindest einem medizinischen Instrument auf eine Sensoreinheit,
b) Ermitteln von zumindest einer Messgröße des zumindest einen medizinischen Instruments durch die Sensoreinheit und eine Steuereinheit,
c) Verarbeitung der zumindest einen Messgröße mittels der Steuereinheit,
d) Aktivierung einer Ausleseeinheit durch die Steuereinheit,
e) Erfassen von Daten von zumindest einem ersten Kennzeichnungselement, des mit der Sensoreinheit in Eingriff stehenden zumindest einen medizinischen Instruments, durch die Ausleseeinheit,
f) Übertragen der von der Ausleseeinheit ermittelten Daten an die Steuereinheit und/oder an ein zweites Kennzeichnungselement,
g) Speichern der Daten und/oder Verfügbarmachung der Daten durch das zweite Kennzeichnungselement zum Austausch von Daten mit zumindest einer externen Sende- und/oder Empfangseinheit.

Dieses Verfahren hat entgegen der bisher üblichen Verfahrensweise den Vorteil, dass der Auslesevorgang bedarfsgerecht auf Basis des oben genannten Verfahrens ausgelöst wird und nicht durch ein manuelles Signal aktiviert wird, das entweder durch das Drücken eines Knopfes oder durch ein anderes externes Signal zur Auslösung kommt.

Des Weiteren können die einzelnen Schritte des Verfahrens auch mehrfach durchgeführt werden und/oder die Schritte des Verfahrens können auch in anderer Reihenfolge angeordnet sein.

Im Übrigen ergeben sich weitere Vorteile aus den obigen Beschreibungen im Zusammenhang mit dem Aufbewahrungs- und/oder Transportbehälter.

In einer weiteren Ausgestaltung des erfindungsgemäßen Verfahrens weist dieses im Schritt b) folgende Schritte auf:
aa) Ermitteln eines von der Sensoreinheit detektierten und mit der zumindest einen Messgröße in Beziehung stehenden Signals,
bb) Umwandeln des Signals in die zumindest eine Messgröße,
cc) Weiterverarbeitung der zumindest einen Messgröße,
dd) Weiterleitung der im Signal enthaltenen Energie an den Energiespeicher.

Diese bevorzugte Ausgestaltung des Verfahrens hat den Vorteil, dass neben der Ermittlung der Messgröße die im Sensorsignal, in Form von Strömen und Spannungen, enthaltene Energie dem Energiespeicher zugeführt werden kann.

In einer weiteren Ausgestaltung des erfindungsgemäßen Verfahrens weist der Schritt cc) folgende Schritte auf:
- Vergleich der zumindest einen Messgröße mit zumindest einem in einem Speicherelement gespeicherten Schwellwert,
- Aktivierung der Ausleseeinheit bei Überschreiten des zumindest einen Schwellwerts durch eine Aktivierungseinheit.

Diese bevorzugte Ausgestaltung des Verfahrens hat den Vorteil, dass durch den Vergleich der zumindest einen Messgröße mit zumindest einem im Speicherelement gespeicherten Schwellwert äußere Einflüsse wie Störsignale, Erschütterungen und auch sonstige Störungen herausgefiltert werden können. Dieser Vorgang ist entscheidend, da hierbei ein Ereignis, das signifikant den Zustand eines medizinischen Instruments verändert, von einem Störereignis unterschieden werden kann.

Ferner ist von Vorteil, dass innerhalb dieses Abgleichs auch mehrere unterschiedliche biologische, physikalische oder chemische Messgrößen mit den jeweiligen im Speicherelement der Steuereinheit gespeicherten Schwellwerten abgeglichen werden können und auch in beliebiger Kombination der ermittelten Messwerte zu einer Aktivierung der Ausleseeinheit führen können.

In einer weiteren Ausgestaltung der Erfindung ist bevorzugt, dass die Sensoreinheit einen Taster oder Schalter aufweist, wobei der Taster oder Schalter durch Erreichen einer vorgegebenen Messgröße elektrische Signale in Form von Strömen und Spannungen an die Steuereinheit übermittelt.

Diese Ausgestaltung hat den Vorteil, dass der Auslesevorgang direkt durch das Erreichen einer vorgegebenen Messgröße ausgelöst wird, die beispielsweise durch das Gewicht zumindest eines medizinischen Instruments festgelegt ist, ohne die Messgröße mit zumindest einem im Speicherelement gespeicherten Schwellwert abzugleichen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: einen erfindungsgemäßen Aufbewahrungs- und/oder Transportbehälter und eine externen Sende- und/oder Empfangseinheit;
- Fig. 2: eine Steuereinheit des Aufbewahrungs- und/oder Transportbehälters mit den darin enthaltenen Einzelelementen;
- Fig. 3: ein zweites Kennzeichnungselement des Aufbewahrungs- und/oder Transportbehälters mit den einzelnen darin integrierten Elementen;
- Fig. 4: eine Darstellung der am Datenausleseprozess beteiligten Elemente des Aufbewahrungs- und/oder Transportbehälters;
- Fig. 5: die für die Verarbeitung, Speicherung und Übermittelung der Daten relevanten Elemente und deren Wechselwirkung;
- Fig. 6: die mit dem Energiespeicher verbundenen und verbindbaren Elemente und eine Prinzipdarstellung der Energiegewinnung; und
- Fig. 7: eine Darstellung des Verfahrens zum Erfassen und Übertragen von Daten medizinischer Instrumente mit dem erfindungsgemäßen Aufbewahrungs- und/oder Transportbehälter anhand eines schematisch dargestellten Flussdiagramms.

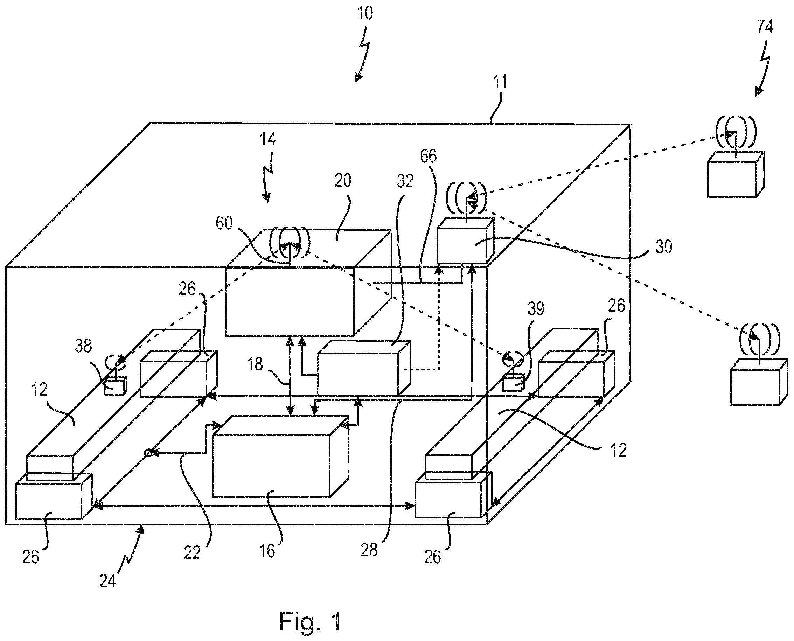

In Fig. 1 ist ein mit dem allgemeinen Bezugszeichen 10 versehener Aufbewahrungs- und/oder Transportbehälter, der einen Behälter 11 zur Aufnahme der medizinischen Instrumente 12 aufweist, schematisch dargestellt. Dieser Aufbewahrungs- und/oder Transportbehälter 10 verfügt ferner über eine Vorrichtung 14 zum Erfassen und Übertragen von Daten der sich im Behälter 11 befindenden medizinischen Instrumente 12. Weitere Details des Aufbewahrungs- und/oder Transportbehälters, sowie der Vorrichtung 14 zum Erfassen und Übertragen von Daten sind in den Figuren 1-6 dargestellt.

Die Vorrichtung 14 zum Erfassen und Übertragen von Daten weist zumindest eine Steuereinheit 16 auf, die mittels einer ersten Schnittstelle 18 mit einer Ausleseeinheit 20 verbunden ist und schematisch in Fig. 1 in Form eines Doppelpfeils dargestellt ist. Ferner weist die Steuereinheit 16 eine Sensorschnittstelle 22 auf, die mit einer Sensoreinheit 24 bestehend aus zumindest einem Sensor 26 in Verbindung steht und ebenfalls durch einen Doppelpfeil in Fig. 1 schematisch gekennzeichnet ist. Ferner weist die Steuereinheit 16 eine erste externe Schnittstelle 28 mit einem zweiten Kennzeichnungselement 30 auf, die durch einen Doppelpfeil schematisch gekennzeichnet ist.

Ferner weist die Vorrichtung 14 zum Erfassen und Übertragen von Daten einen Energiespeicher 32 auf, der zumindest die Steuereinheit 16 und die Ausleseeinheit 20 mit Energie versorgt. Der Energiespeicher 32 ist bevorzugt durch eine elektrochemische Zelle oder durch ein kapazitives Speicherelement ausgestaltet.

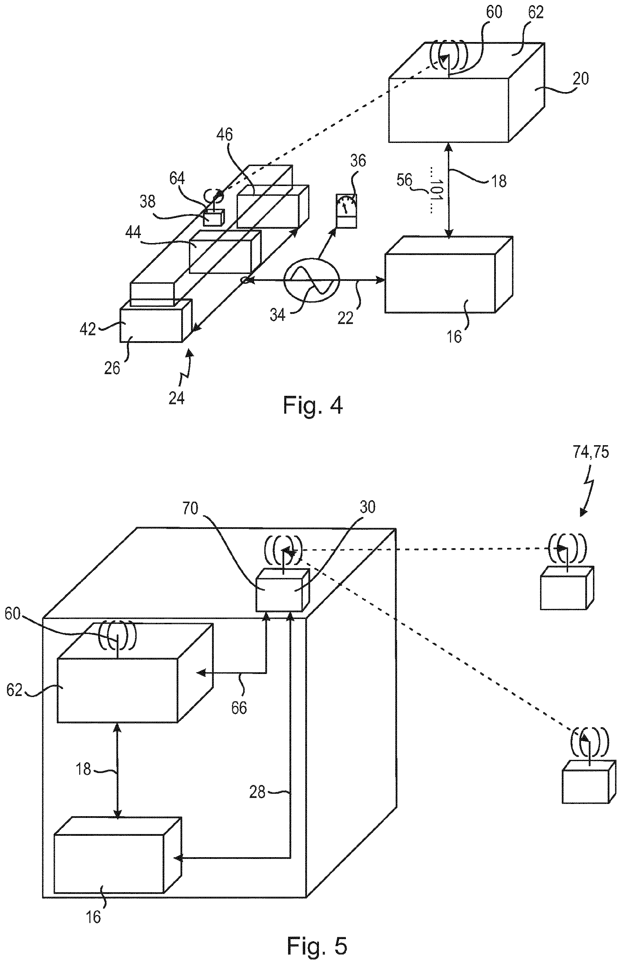

Die Steuereinheit 16 empfängt über die Sensorschnittstelle 22 Signale 34 (Fig. 4) von der Sensoreinheit 24, die in der Steuereinheit 16 weiter verarbeitet werden. Die Signale 34 werden hervorgerufen durch Messungen von zumindest einer Messgröße 36 (Fig. 4) an den mit der Sensoreinheit 24 in Wechselwirkung stehenden medizinischen Instrumenten 12.

Die medizinischen Instrumente 12 ihrerseits sind mit Kennzeichnungselementen 38 versehen, die mit der Ausleseeinheit 20 drahtlos zum Austausch von Daten verbindbar sind.

Demnach kann das durch eine Messung der Sensoreinheit 24 an den medizinischen Instrumenten 12 erzeugte Signal 34 unter anderem dazu verwendet werden, eine Verbindung zwischen den Kennzeichnungselementen 38 und der Ausleseeinheit 20 herzustellen und damit einen Datenaustausch zwischen der Ausleseeinheit 20 und dem ersten Kennzeichnungselement 38 in Gang zu setzen. Die Verwendung der Signale 34 der Sensoreinheit 24 beschränkt sich nicht nur auf das Ingangsetzen des Ausleseprozesses zwischen der Ausleseeinheit 20 und dem zumindest einen ersten Kennzeichnungselement 38, sondern kann allgemein zur Aktivierung bzw. zum Triggern der Steuereinheit 16 und/oder dem zweiten Kennzeichnungselement 30 verwendet werden, was die durch den Energiespeicher 32 bereitzustellende Energie zur Versorgung von zumindest der Steuereinheit 16 und der Ausleseeinheit 20 auf ein Mindestmaß reduziert.

Es versteht sich, dass das zumindest eine erste Kennzeichnungselement 38 individuell den medizinischen Instrumenten 12 zugeordnet ist, um eine eindeutige Kennzeichnung zu erreichen. Ferner können weiteren medizinischen Instrumenten 12 auch weitere Kennzeichnungselemente 39 zugeordnet sein.

In einer bevorzugten Ausgestaltung weist die Sensoreinheit 24 zumindest einen Gewichtssensor 42 (Fig. 4) auf, der das Gewicht der medizinischen Instrumente 12 erfasst. Darüber hinaus kann die Sensoreinheit 24, wie in Fig. 4 dargestellt, noch über weitere Sensoren 44 zur Ermittlung weiterer physikalischer, biologischer und/oder chemischer Parameter der medizinischen Instrument 12 und/oder der Umgebung, wie z.B. der Temperatur, der Luftfeuchte, des pH-Werts, der Magnetisierung, und/oder der Radioaktivität verfügen. Auch die hierbei ermittelten Signale 34 können unter anderem ebenfalls zum Triggern der beschriebenen Gerätefunktion, aber auch für andere Funktionen, die auf den durch die Sensoreinheit 24 ermittelten Messgrößen 36 beruhen, herangezogen werden.

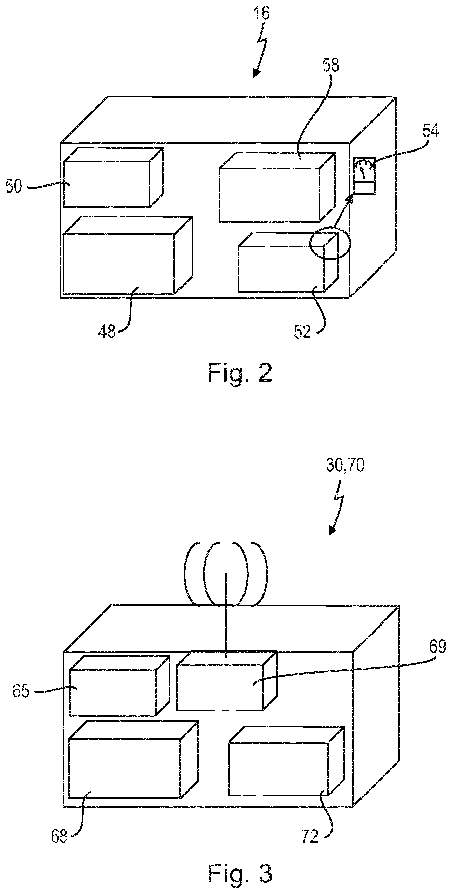

In einer weiteren bevorzugten Ausgestaltung ist der Gewichtssensor 42 mittels eines piezoelektrischen Elements 46 ausgebildet, der durch den Auflagedruck der medizinischen Instrumente 12 Signale 34, in Form von Strömen und Spannungen, an eine in der Steuereinheit 16 integrierte Übermittlungseinheit 48 (Fig. 2) weiterleitet. Wie ebenfalls in Fig. 2 dargestellt, werden die Signale 34 in der Steuereinheit 16 durch die Übermittlungseinheit 48, das Rechnerelement 50 und das Speicherelement 52 weiterverarbeitet. Hierbei wird durch das Zusammenwirken der Übermittlungseinheit 48, des Rechnerelements 50 und des Speicherelements 52 das Signal 34 in eine Messgröße 36 umgewandelt. Diese Messgröße 36 kann im Anschluss mit zumindest einem im Speicherelement 52 gespeicherten Referenz- bzw. Schwellwert 54 verglichen werden.

In weiteren Ausführungsformen kann die Sensoreinheit 24 einen Taster oder Schalter aufweisen, der durch das Erreichen einer Messgröße 36, Signale 34 in Form von Strömen und Spannungen, an die in der Steuereinheit 16 integrierte Übermittlungseinheit 48 übermittelt.

In einer weiteren Ausführungsform kann der Taster oder Schalter durch den durch das Auflegen der medizinischen Instrumente 12 vermittelten Auflagedruck eine vorgegebene Stellung einnehmen, in der Signale 34 in Form von Strömen und Spannungen an die Steuereinheit 16 übermittelt werden.

Entspricht nun der Schwellwert 54 bei einer Gewichtsmessung durch den Gewichtssensor 42 gerade dem Gewicht eines medizinischen Instruments 12, kann durch den Abgleich der ermittelten Messgröße 36 und des Schwellwerts 54 das Vorhandensein bzw. Nichtvorhandensein eines medizinischen Instruments 12 ermittelt werden. Somit kann durch das Entfernen bzw. Hinzufügen zumindest eines medizinischen Instruments 12, durch das Zusammenwirken der Übermittlungseinheit 48, des Rechnerelements 50 und des Speicherelements 52, ein Triggersignal 56 (Fig. 4) erzeugt werden, das über eine Aktivierungseinheit 58 zumindest an die Ausleseeinheit 20 mittels der ersten Schnittstelle 18 weitergeleitet wird.

Das Gleiche gilt natürlich auch für jeden physikalischen, chemischen und/oder biologischen Parameter, dessen Änderung durch eine Messung an den medizinischen Instrumenten 12 ermittelt werden kann.

Es versteht sich, dass sowohl diese als auch jede andere physikalische, biologische und/oder chemische Messgröße 36, die durch die Sensoreinheit 24 ermittelt und in der Steuereinheit 16 weiterverarbeitet wird, entweder in der Steuereinheit 16 oder im integrierten Speicherelement 52 gespeichert oder mittels der Übermittlungseinheit 48 und einer ersten externen Schnittstelle 28 an das zweite Kennzeichnungselement 30 übermittelt werden kann.

Der Auslesevorgang an dem zumindest einen ersten Kennzeichnungselement 38 kann nun aktiviert durch ein Triggersignal 56, wie zu sehen in Fig. 4, von der Ausleseeinheit 20 mittels eines Sende- und/oder Empfangselements 60, vollzogen werden.

In einer weiteren bevorzugten Ausführungsform ist die Vorrichtung 14 solange inaktiv, d.h. befindet sich in einem Stand-by-Modus, bis die Steuereinheit 16 das Triggersignal 56 erzeugt und weiterleitet.

Wie in Fig. 5 zu sehen ist, ist es ferner bevorzugt, die Ausleseeinheit 20 als aktive RFID- oder RFID-UHF-Ausleseeinheit 62 mit aktivem Sende- und/oder Empfangselements 60 auszubilden, das drahtlos mit dem zumindest einen ersten Kennzeichnungselement 38, das seinerseits bevorzugt als passives RFID-Tag 64 ausgebildet ist, Daten austauscht. Zusätzlich weist das zweite Kennzeichnungselement 30 ein Energiespeicherelement 65 zur Energieversorgung auf.

Es versteht sich, dass durch die Kommunikation mit der Ausleseeinheit 20 und dem zumindest einen ersten Kennzeichnungselement 38 sowohl Daten von der Ausleseeinheit 20 auf das erste Kennzeichnungselement 38 übertragen werden können, als auch Daten von dem ersten Kennzeichnungselement 38 über die Ausleseeinheit 20 ausgelesen werden können. Der Datentransfer der Ausleseeinheit 20 wird somit mittels einer ersten Schnittstelle 18, zwischen der Steuereinheit 16 und der Ausleseeinheit 20 und über eine zweite externe Schnittstelle 66, zwischen der Ausleseeinheit und dem zweiten Kennzeichnungselement 30 sichergestellt.

Die von dem zumindest einen ersten Kennzeichnungselement 38 ermittelten Daten können ferner über die erste externe Schnittstelle 28 (Fig. 1 und Fig. 5), die die Steuereinheit 16 mit dem zweiten Kennzeichnungselement 30 verbindet oder über die zweite externe Schnittstelle 66, die die Ausleseeinheit 20 mit dem zweiten Kennzeichnungselement 30 verbindet, an das im zweiten Kennzeichnungselement 30 integrierte Datenspeicherelement 68 übermittelt werden.

Das zweite Kennzeichnungselement 30, das bevorzugt mit aktivem Sender 69 als aktiver RFID-UHF-Transponder 70 ausgestaltet ist, sendet bzw. empfängt Daten mittels eines zweiten aktiven Sende- und/oder Empfangselements 72 an bzw. von einer außerhalb der Vorrichtung 14 gelegenen externen Sende- und/oder Empfangseinheit 74, die bevorzugt in Form einer aktiven RFID-UHF-Infrastruktur 75 ausgebildet ist.

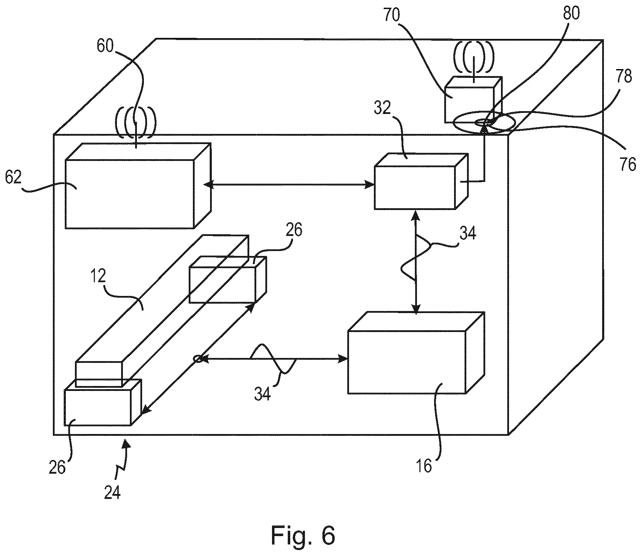

In einer weiteren bevorzugten Ausführungsform der Erfindung werden die durch die Wechselwirkung der medizinischen Instrumente 12 mit der Sensoreinheit 24 erzeugten Signale 34, wie in Fig. 6 dargestellt, zur Energiegewinnung genutzt. Hierbei kann innerhalb einer weiteren bevorzugten Ausführungsform, bei der die Sensoreinheit 24 aus zumindest einem piezoelektrischen Element 46 besteht, die durch die Gewichtsmessung des medizinischen Instruments 12 erzeugten und aus Strömen und Spannungen bestehenden Signale 34 dazu genutzt werden, die darin enthaltene Energie über die Steuereinheit 16 an den Energiespeicher 32 zu leiten.

Entspricht nun das zu einer Messgröße 36 korrespondierende Signal 34 nicht dem im Speicherelement 52 der Steuereinheit 16 gespeicherten Schwellwerts 54, sondern ist kleiner als derselbe, wird lediglich die gesamte in dem Signal 34 gespeicherte Energie über die in der Steuereinheit integrierte Übermittlungseinheit 48 direkt an den Energiespeicher 32 übermittelt.

Die hierbei auftretende Größenordnung der Signale 34 korrespondiert zu Ereignissen, bei denen die piezoelektrischen Elemente 46 beispielsweise durch Erschütterungen der medizinischen Instrumente oder auch durch andere Störeinflüsse bedingt, ein Signal 34 detektieren, ohne dass ein medizinisches Instrument 12 als Ganzes von der Sensoreinheit 24 bzw. dem piezoelektrischen Element 46 direkt entfernt wird. Diese Differenzierung ist ferner notwendig, um etwaige Störsignale, die den Prozessablauf empfindlich stören können, herauszufiltern.

Das Gleiche gilt natürlich auch für jeden physikalischen, chemischen und/oder biologischen Parameter, dessen Änderung durch eine Messung an den medizinischen Instrumenten ermittelt werden kann.

Darüber hinaus kann die gesamte im Signal 34 enthaltene Energie, die zur Ermittlung der Messgröße 36 nicht benötigt wird, direkt an den Energiespeicher 32 durch die Übermittlungseinheit 48 weitergeleitet werden.

Die beschriebene Übermittlung der Energie durch die Steuereinheit 16 an den Energiespeicher 32 kann analog auch dann angewendet werden, wenn die zu den Messgrößen 36 korrespondierenden Signale 34 die im Speicherelement 52 gespeicherten Schwellwerte 54 erreichen bzw. überschreiten.

Dieser Vorgang ist nochmals zur Illustration in Fig. 6 dargestellt. Hierbei ist zu sehen, dass der Energiespeicher 32 zumindest die Steuereinheit 16 und die Ausleseeinheit 20, die bevorzugt eine RFID-Ausleseeinheit 62 ist, mit Energie versorgt. Wie bereits beschrieben, kann die Sensoreinheit 24 durch die mit den medizinischen Instrumenten 12 erzeugte Wechselwirkung Signale 34 in Form von Strömen und Spannungen generieren, wobei die in den Signalen 34 enthaltene Energie über die Steuereinheit 16 an den Energiespeicher 32 geleitet werden kann.

In einer weiteren bevorzugten Ausführungsform, ist das zweite Kennzeichnungselement 30 in Form eines aktiven RFID-Transponders 70 zur alternativen und/oder zusätzlichen Energieversorgung mit dem in der Vorrichtung 14 integrierten Energiespeicher 32 über eine Energietransferschnittstelle 76 (Fig. 6) verbunden. Diese Schnittstelle ist in einer ersten bevorzugten Ausführungsform als kapazitive und/oder induktive Schnittstelle 78 zwischen dem Energiespeicher 32 und dem zweiten Kennzeichnungselement 30 realisiert.

In einer weiteren bevorzugten Ausführungsform ist die Energietransferschnittstelle 76 mittels eines metallischen Kontakts 80 zwischen dem Energiespeicher 32 und dem zweiten Kennzeichnungselement 30 realisiert.

Die beschriebene Form der Energieversorgung mittels eines zentralen Energiespeichers 32 ist vorteilhaft hinsichtlich der Langlebigkeit und damit der Wirtschaftlichkeit des beschriebenen Aufbewahrungsbehälters 10. Hierbei ist von Vorteil, dass im Wesentlichen sowohl die Aktivierung der Energieversorgung durch Signale 34 der Sensoreinheit 24 und auch die Energiegewinnung über die in den benannten Signalen 34 enthaltenen Energie, die mittels der Steuereinheit 16 an den Energiespeicher 32 transferiert wird, zentral über einen Energiespeicher 32 der Vorrichtung 14 abgewickelt werden kann und dadurch ein zusätzliches Energiespeicherelement 65 im zweiten Kennzeichnungselement 30 nicht notwendig ist bzw. die Energiereserven des Energiespeicherelements 65 geschont werden.

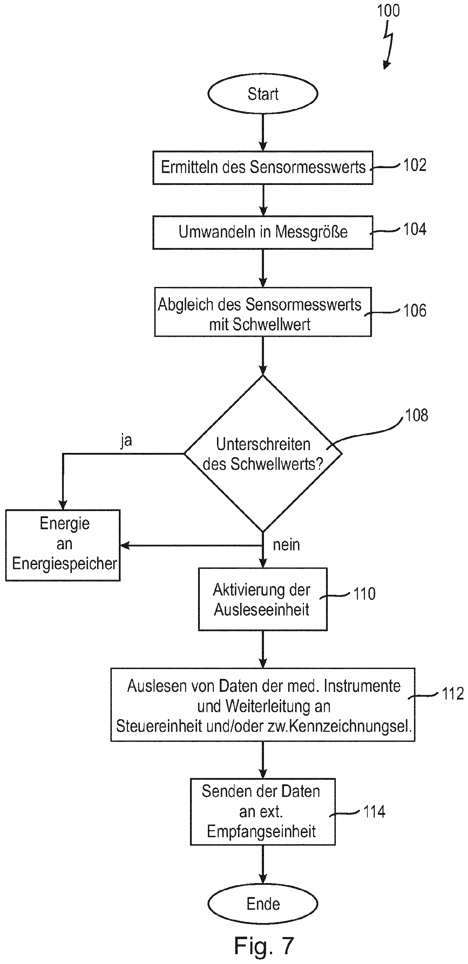

Im Folgenden wird nochmals das Verfahren, gekennzeichnet mit der allgemeinen Bezugsziffer 100, zur Aktivierung des Ausleseprozesses mittels einer Ausleseeinheit 20 anhand eines in Fig. 7 dargestellten Flussdiagramms nachvollzogen und kurz zusammengefasst.

Das Verfahren beginnt mit der Ermittelung des Sensormesswerts 102 durch die Steuereinheit 16, wobei im Folgenden Schritt 104 das ermittelte Signal 34 am Sensor mittels der Steuereinheit 16 in eine Messgröße 36 umgewandet wird. Nach Abgleich 106 der Messgröße 36 mit zumindest einem im Speicherelement 52 der Steuereinheit 16 gespeicherten Schwellwerts 54, wird durch das Zusammenwirken des Rechnerelements 50 und des Speicherelements 52 ein Unterscheidungsschritt 108 vollzogen. Unterschreitet nun die ermittelte Messgröße 36 den im Speicherelement 52 gespeicherten Schwellwert 54, wird die in den Signalen 34 enthaltene Energie direkt an den Energiespeicher 32 weitergeleitet.

Erreicht oder überschreitet die ermittelte Messgröße 36 den im Speicherelement 52 gespeicherten Schwellwert 54, wird durch einen weiteren Prozessschritt 110 die Aktivierungseinheit 58 den durch die Ausleseeinheit 20 vermittelten Ausleseprozess aktivieren. Die überschüssige Energie wird dem Energiespeicher 32 zugeführt.

Im folgenden Prozessschritt 112, werden die Daten, die in dem zumindest einen ersten Kennzeichnungselement 38 gespeichert sind, über die Ausleseeinheit 20 an die Steuereinheit 16 und/oder das zweite Kennzeichnungselement 30 übermittelt.

Im Folgenden Prozessschritt 114 werden die ermittelten Daten in der Steuereinheit 16 und/oder dem zweiten Kennzeichnungselement 30 gespeichert und/oder an eine externe Empfangseinheit 74, welche bevorzugt in Form einer aktiven RFID-UHF-Infrastruktur 75 ausgebildet ist, weitergeleitet.

## Patentansprüche

1. Aufbewahrungs- und/oder Transportbehälter für medizinische Instrumente, mit einem Behälter (11) zur Aufnahme der medizinische Instrumente und einer Vorrichtung (14) zum Erfassen und Übertragen von Daten der medizinischen Instrumente (12), die eine Steuereinheit (16), eine Ausleseeinheit (20) und zumindest ein an zumindest einem medizinischen Instrument (12) angebrachtes erstes Kennzeichnungselement (38) aufweist, wobei die Steuereinheit (16) mit der Ausleseeinheit (20) in Verbindung steht und mit der Ausleseeinheit (20), ausgelöst durch Signale (34), Daten austauscht und diese weiterverarbeitet, speichert und/oder an ein an dem Behälter (11) angebrachtes zweites Kennzeichnungselement (30) weiterleitet, wobei die Ausleseeinheit (20) Daten mit dem zumindest einen ersten Kennzeichnungselement (38) austauscht und an die Steuereinheit (16) weiterleitet, **dadurch gekennzeichnet, dass** die Vorrichtung (14) eine Sensoreinheit (24) aufweist, die das zumindest eine medizinische Instrument (12) durch eine an ihm durchgeführte Messung zumindest einer Messgröße (36) erfasst, und mit der Steuereinheit (16) Signale (34) austauscht, wobei die ausgetauschten Signale (34) den Austausch von Daten zwischen der Ausleseeinheit (20) und dem zumindest einen ersten Kennzeichnungselement (38) in Gang setzt, wobei die Ausleseeinheit (20) die ausgetauschten Daten an die Steuereinheit (16) übermittelt.

2. Aufbewahrungs- und/oder Transportbehälter nach Anspruch 1, **dadurch gekennzeichnet, dass** die Steuereinheit (16) zumindest ein Speicherelement (52), zumindest ein Rechnerelement (50), zumindest eine Übermittlungseinheit (48) und zumindest eine Aktivierungseinheit (58) aufweist, wobei die Aktivierungseinheit (58) mit dem Speicherelement (52), dem Rechnerelement (50), der Übermittlungseinheit (48) und der Ausleseeinheit (20), zur Aktivierung von Auslese-, Verarbeitungs- und/oder Übermittlungsvorgängen von Daten, verbunden ist.

3. Aufbewahrungs- und/oder Transportbehälter nach Anspruch 1 oder 2, **dadurch gekennzeichnet, dass** das zweite Kennzeichnungselement (30) zumindest ein Datenspeicherelement (68) und zumindest ein Sende- und/oder Empfangselement (72) zum Austausch von Daten mit einer externen Sende- und/oder Empfangseinheit (74) aufweist.

4. Aufbewahrungs- und/oder Transportbehälter nach einem der Ansprüche 1 bis 3, **dadurch gekennzeichnet, dass** das zweite Kennzeichnungselement (30) zumindest einen aktiven Sender (69) aufweist.

5. Aufbewahrungs- und/oder Transportbehälter nach einem der Ansprüche 1 bis 4, **dadurch gekennzeichnet, dass** das zweite Kennzeichnungselement (30) zumindest einen aktiven RFID-Transponder (70) aufweist.

6. Aufbewahrungs- und/oder Transportbehälter nach einem der Ansprüche 1 bis 5, **dadurch gekennzeichnet, dass** die Ausleseeinheit (20) zumindest ein Sende- und/oder Empfangselement (60) aufweist, das mit dem zumindest einen ersten Kennzeichnungselement (38) drahtlos Daten austauscht.

7. Aufbewahrungs- und/oder Transportbehälter nach einem der Ansprüche 1 bis 6, **dadurch gekennzeichnet, dass** die Ausleseeinheit (20) mit dem zweiten Kennzeichnungselement (30) zumindest eine Verbindung (66) zum Austausch von Daten aufweist.

8. Aufbewahrungs- und/oder Transportbehälter nach einem der Ansprüche 1 bis 7, **dadurch gekennzeichnet, dass** die Sensoreinheit (24) zumindest einen Gewichtssensor (42) aufweist, der die zumindest eine Messgröße (36) in Form des Gewichts von zumindest einem der medizinischen Instrumente (12) erfasst.

9. Aufbewahrungs- und/oder Transportbehälter nach Anspruch 8, **dadurch gekennzeichnet, dass** der Gewichtssensor (42) ein piezoelektrisches Element (46) ist, wobei dieses elektrische Signale (34) in Form von Strömen und Spannungen an die Steuereinheit (16) übermittelt.

10. Aufbewahrungs- und/oder Transportbehälter nach einem der Ansprüche 1 bis 9, **dadurch gekennzeichnet, dass** die Vorrichtung einen Energiespeicher (32) aufweist, wobei die Sensoreinheit (24) über die Steuereinheit (16) mit dem Energiespeicher (32) verbunden ist.

11. Aufbewahrungs- und/oder Transportbehälter nach Anspruch 10, **dadurch gekennzeichnet, dass** das zweite Kennzeichnungselement (30) mit dem Energiespeicher (32) verbindbar ist.

12. Aufbewahrungs- und/oder Transportbehälter nach einem der Ansprüche 1 bis 7, **dadurch gekennzeichnet, dass** die Sensoreinheit (24) einen Taster oder Schalter aufweist, wobei der Taster oder Schalter durch Erreichen einer vorgegebenen Messgröße (36) elektrische Signale (34) in Form von Strömen und Spannungen an die Steuereinheit (16) übermittelt.

13. Verfahren zum Erfassen und Übertragen von Daten von zumindest einem medizinischen Instrument (12) in einem Aufbewahrungs- und/oder Transportbehälter, insbesondere einem Aufbewahrungs- und/oder Transportbehälter nach einem der Ansprüche 1 bis 12, das folgende Schritte aufweist:
a) Auflegen von zumindest einem medizinischen Instrument (12) auf eine Sensoreinheit (24),
b) Ermitteln von zumindest einer Messgröße (36) des zumindest einen medizinischen Instruments (12) durch die Sensoreinheit (24) und eine Steuereinheit (16),
c) Verarbeitung der zumindest einen Messgröße (36) mittels der Steuereinheit (16),
d) Aktivierung einer Ausleseeinheit (20) durch die Steuereinheit (16),
e) Erfassen von Daten von zumindest einem ersten Kennzeichnungselement (38), des mit der Sensoreinheit (24) in Eingriff stehenden zumindest einen medizinischen Instruments (12), durch die Ausleseeinheit (20),
f) Übertragen der von der Ausleseeinheit (20) ermittelten Daten an die Steuereinheit (16) und/oder an ein zweites Kennzeichnungselement (30),
g) Speichern der Daten und/oder Verfügbarmachung der Daten durch das zweite Kennzeichnungselement (30) zum Austausch der Daten mit zumindest einer externen Sende und/oder Empfangseinheit (74).

14. Verfahren nach Anspruch 13, **dadurch gekennzeichnet, dass** Schritt b) folgende Schritte aufweist:
aa) Ermitteln eines von der Sensoreinheit (16) detektierten und mit der zumindest einen Messgröße (36) in Beziehung stehenden Signals (34),
bb) Umwandeln des Signals (34) in die zumindest eine Messgröße (36),
cc) Weiterverarbeitung der zumindest einen Messgröße (36),
dd) Weiterleitung der im Signal (34) enthaltenen Energie an den Energiespeicher (32).

15. Verfahren nach Anspruch 13 oder 14, **dadurch gekennzeichnet, dass** Schritt cc) folgende Schritte aufweist:
- Vergleich der zumindest einen Messgröße (36) mit zumindest einem in einem Speicherelement (52) gespeicherten Schwellwert (54),
- Aktivierung der Ausleseeinheit (20) bei Überschreiten des zumindest einen Schwellwerts (54) durch eine Aktivierungseinheit (58).

## Claims

1. Storage and/or transport holder for medical instruments, comprising a container (11) for holding the medical instruments and comprising an apparatus (14) for capturing and transmitting data from the medical instruments (12), the apparatus comprising a control unit (16), a reader (20) and at least one first identification element (38) attached to at least one medical instrument (12), wherein the control unit (16) is connected to the reader (20) and, actuated by signals (34), exchanges data with the reader (20), and processes, stores and/or passes to a second identification element (30) attached to the container (11) said data, wherein the reader (20) exchanges data with the at least one first identification element (38) and passes said data to the control unit (16), **characterized in that** the apparatus (14) comprises a sensor unit (24) which detects the at least one medical instrument (12) by a measurement performed thereon of at least one measurement quantity (36), and exchanges signals (34) with the control unit (16), wherein the exchanged signals (34) initiate the exchange of data between the reader (20) and the at least one first identification element (38), wherein the reader (20) transfers the exchanged data to the control unit (16).

2. Storage and/or transport holder as claimed in claim 1, **characterized in that** the control unit (16) comprises at least one memory element (52), at least one processing element (50), at least one transfer unit (48) and at least one activation unit (58), wherein the activation unit (58) is connected to the memory element (52), the processing element (50), the transfer unit (48) and the reader (20) for the purpose of activating processes for reading, processing and/or transferring data.

3. Storage and/or transport holder as claimed in claim 1 or 2, **characterized in that** the second identification element (30) comprises at least one data storage element (68) and at least one transceiver element (72) for exchanging data with an external transceiver unit (74).

4. Storage and/or transport holder as claimed in any of claims 1 to 3, **characterized in that** the second identification element (30) comprises at least one active transmitter (69).

5. Storage and/or transport holder as claimed in any of claims 1 to 4, **characterized in that** the second identification element (30) comprises at least one active RFID transponder (70).

6. Storage and/or transport holder as claimed in any of claims 1 to 5, **characterized in that** the reader (20) comprises at least one transceiver element (60), which exchanges data wirelessly with the at least one first identification element (38).

7. Storage and/or transport holder as claimed in any of claims 1 to 6, **characterized in that** the reader (20) comprises at least one connection (66) to the second identification element (30) for the purpose of exchanging data.

8. Storage and/or transport holder as claimed in any of claims 1 to 7, **characterized in that** the sensor unit (24) comprises at least one weight sensor (42), which detects the at least one measurement quantity (36) in the form of the weight of at least one of the medical instruments (12).

9. Storage and/or transport holder as claimed in claim 8, **characterized in that** the weight sensor (42) is a piezoelectric element (46), wherein said piezoelectric element transfers electrical signals (34) in the form of currents and voltages to the control unit (16).

10. Storage and/or transport holder as claimed in any of claims 1 to 9, **characterized in that** the apparatus comprises an energy storage device (32), wherein the sensor unit (24) is connected to the energy storage device (32) via the control unit (16).

11. Storage and/or transport holder as claimed in claim 10, **characterized in that** the second identification element (30) can be connected to the energy storage device (32).

12. Storage and/or transport holder as claimed in any of claims 1 to 7, **characterized in that** the sensor unit (24) comprises a pushbutton or switch, wherein by virtue of reaching a predetermined measurement quantity (36), the pushbutton or switch transfers electrical signals (34) in the form of currents and voltages to the control unit (16).

13. Method for capturing and transmitting data from at least one medical instrument (12) in a storage and/or transport holder, in particular a storage and/or transport holder as claimed in any of claims 1 to 12, which method comprises the following steps:
a) placing at least one medical instrument (12) onto a sensor unit (24),
b) the sensor unit (24) and a control unit (16) determining at least one measurement quantity (36) of the at least one medical instrument (12),
c) the control unit (16) processing the at least one measurement quantity (36),
d) the control unit (16) activating a reader (20),
e) the reader (20) capturing data from at least one first identification element (38) of the at least one medical instrument (12) engaging with the sensor unit (24),
f) transmitting the data determined by the reader (20) to the control unit (16) and/or to a second identification element (30),
g) the second identification element (30) storing the data and/or making the data available in order to exchange the data with at least one external transceiver unit (74).

14. Method as claimed in claim 13, **characterized in that** step b) comprises the following steps:
aa) determining a signal (34) detected by the sensor unit (16) and related to the at least one measurement quantity (36),
bb) converting the signal (34) into the at least one measurement quantity (36),
cc) processing the at least one measurement quantity (36),
dd) transferring the energy contained in the signal (34) to the energy storage device (32).

15. Method as claimed in claim 13 or 14, **characterized in that** step cc) comprises the following steps:
- comparing the at least one measurement quantity (36) with at least one threshold value (54) stored in a memory element (52),
- an activation unit (58) activating the reader (20) when the at least one threshold value (54) is exceeded.

## Revendications

1. Réceptacle de rangement et/ou de transport pour des instruments médicaux, avec un réceptacle (11), lequel est destiné au logement des instruments médicaux, et avec un dispositif (14), lequel est destiné à la saisie et au transfert de données relatives aux instruments médicaux (12) et lequel présente une unité de commande (16), une unité de lecture (20) et tout au moins un premier élément de désignation (38) mis en place sur tout au moins un instrument médical (12), dans lequel l'unité de commande (16) se trouve en liaison avec l'unité de lecture (20) et échange des données avec l'unité de lecture (20), déclenchée par des signaux (34), et les traite, les enregistre et/ou les retransmet vers un deuxième élément de désignation (30) mis en place au niveau du réceptacle (11), dans lequel l'unité de lecture (20) échange des données avec le tout au moins un premier élément de désignation (38) et les retransmet à l'unité de commande (16), **caractérisé en ce que** le dispositif (14) présente une unité de capteur (24), laquelle détecte le tout au moins un instrument médical (12) en effectuant sur ce dernier une mesure de tout au moins une grandeur de mesure (36), et échange des signaux (34) avec l'unité de commande (16), dans lequel les signaux (34) échangés déclenchent l'échange de données entre l'unité de lecture (20) et le tout au moins un premier élément de désignation (38), dans lequel l'unité de lecture (20) transmet les données échangées à l'unité de commande (16).

2. Réceptacle de rangement et/ou de transport selon la revendication 1, **caractérisé en ce que** l'unité de commande (16) présente tout au moins un élément de mémoire (52), tout au moins un élément de calcul (50), tout au moins une unité de transmission (48) et tout au moins une unité d'activation (58), dans lequel l'unité d'activation (58) est reliée à l'élément de mémoire (52), à l'élément de calcul (50), à l'unité de transmission (48) et à l'unité de lecture (20) en vue de l'activation des processus de lecture, de traitement et/ou de transfert des données.

3. Réceptacle de rangement et/ou de transport selon la revendication 1 ou 2, **caractérisé en ce que** le deuxième élément de désignation (30) présente tout au moins un élément d'enregistrement des données (68) et tout au moins un élément de transmission et/ou de réception (72), en vue de l'échange des données avec une unité de transmission et/ou de réception (74) externe.

4. Réceptacle de rangement et/ou de transport selon l'une des revendications 1 à 3, **caractérisé en ce que** le deuxième élément de désignation (30) présente tout au moins un émetteur (69) actif.

5. Réceptacle de rangement et/ou de transport selon l'une des revendications 1 à 4, **caractérisé en ce que** le deuxième élément de désignation (30) présente tout au moins un transpondeur RFID (70) actif.

6. Réceptacle de rangement et/ou de transport selon l'une des revendications 1 à 5, **caractérisé en ce que** l'unité de lecture (20) présente tout au moins un élément de transmission et/ou de réception (60) qui réalise un échange sans fil des données avec le tout au moins un premier élément de désignation (38).

7. Réceptacle de rangement et/ou de transport selon l'une des revendications 1 à 6, **caractérisé en ce que** l'unité de lecture (20) présente tout au moins une liaison (66) avec le deuxième élément de désignation (30) en vue de l'échange des données.

8. Réceptacle de rangement et/ou de transport selon l'une des revendications 1 à 7, **caractérisé en ce que** l'unité de capteur (24) présente tout au moins un capteur de poids (42), lequel détecte la tout au moins une grandeur de mesure (36) sous la forme du poids de tout au moins l'un des instruments médicaux (12).

9. Réceptacle de rangement et/ou de transport selon la revendication 8, **caractérisé en ce que** le capteur de poids (42) est un élément piézoélectrique (46), dans lequel ce dernier transmet des signaux (34) électriques à l'unité de commande (16) sous la forme de courants et de tensions.

10. Réceptacle de rangement et/ou de transport selon l'une des revendications 1 à 9, **caractérisé en ce que** le dispositif présente un accumulateur d'énergie (32), dans lequel l'unité de capteur (24) est reliée à l'accumulateur d'énergie (32) par l'intermédiaire de l'unité de commande (16).

11. Réceptacle de rangement et/ou de transport selon la revendication 10, **caractérisé en ce que** le deuxième élément de désignation (30) peut être relié à l'accumulateur d'énergie (32).

12. Réceptacle de rangement et/ou de transport selon l'une des revendications 1 à 7, **caractérisé en ce que** l'unité de capteur (24) présente une touche ou un commutateur, dans lequel la touche ou le commutateur transmet des signaux (34) électriques à l'unité de commande (16) sous la forme de courants et de tensions, après avoir atteint une grandeur de mesure (36) prédéfinie.

13. Procédé destiné à la saisie et au transfert de données de tout au moins un instrument médical (12) dans un réceptacle de rangement et/ou de transport, en particulier dans un réceptacle de rangement et/ou de transport selon l'une des revendications 1 à 12 qui présente les phases suivantes :
a) la pose de tout au moins un instrument médical (12) sur une unité de capteur (24) ;
b) la détermination de tout au moins une grandeur de mesure (36) du tout au moins un instrument médical (12) par l'intermédiaire de l'unité de capteur (24) et d'une unité de commande (16) ;
c) le traitement de la tout au moins une grandeur de mesure (36) au moyen de l'unité de commande (16) ;
d) l'activation d'une unité de lecture (20) par l'unité de commande (16) ;
e) la saisie de données par tout au moins un premier élément de désignation (38) du tout au moins un instrument médical (12) qui se trouve en prise avec l'unité de capteur (24), par l'intermédiaire de l'unité de lecture (20) ;
f) le transfert des données déterminées par l'unité de lecture (20) à l'unité de commande (16) et/ou à un deuxième élément de désignation (30) ;
g) l'enregistrement des données et/ou la mise à disposition des données par l'intermédiaire du deuxième élément de désignation (30) en vue de l'échange des données avec tout au moins une unité de transmission et/ou de réception (74) externe.

14. Procédé selon la revendication 13, **caractérisé en ce que** la phase b) présente les phases suivantes :
aa) la détermination d'un signal (34), lequel est détecté par l'unité de capteur (16) et lequel se trouve en relation avec la tout au moins une grandeur de mesure (36) ;
bb) la conversion du signal (34) dans la tout au moins une grandeur de mesure (36) ;
cc) le traitement de la tout au moins une grandeur de mesure (36) ;
dd) la retransmission de l'énergie contenue dans le signal (34) vers l'accumulateur d'énergie (32).

15. Procédé selon la revendication 13 ou 14, **caractérisé en ce que** la phase cc) présente les phases suivantes :
- la comparaison de la tout au moins une grandeur de mesure (36) avec tout au moins une valeur de seuil (54) enregistrée dans un élément de mémoire (52) ;
- l'activation de l'unité de lecture (20) en cas de dépassement de la tout au moins une valeur de seuil (54) par une unité d'activation (58).
